Europäisches Patentamt

European Patent Office

Office européen des brevets

(19)

(11) Veröffentlichungsnummer: **0 289 822 B1**

(12) **EUROPÄISCHE PATENTSCHRIFT**

(45) Veröffentlichungstag der Patentschrift: 27.11.91

(21) Anmeldenummer: 88105933.1

(22) Anmeldetag: 14.04.88

(51) Int. Cl.⁵: **C07C 51/235**, C12P 7/40, C12P 7/52

(54) Verfahren zur Herstellung von Carbonsaüren durch mikrobielle Oxidation von Alkoholen.

(30) Priorität: 24.04.87 DE 3713668

(43) Veröffentlichungstag der Anmeldung:
09.11.88 Patentblatt 88/45

(45) Bekanntmachung des Hinweises auf die
Patenterteilung:
27.11.91 Patentblatt 91/48

(84) Benannte Vertragsstaaten:
CH DE ES FR GB IT LI NL

(56) Entgegenhaltungen:
DE-A- 2 853 847
FR-A- 2 587 361
US-A- 4 400 468

PATENT ABSTRACTS OF JAPAN, Band 8, Nr.
232 (C-248)[1669] 25. Oktober 1984; & JP - A -
59 113 891 (MITSUBISHI RAYON) 30.06.1984

JOURNAL OF ORGANIC CHEMISTRY, Band
47, Nr. 12, 4. Juni 1982, Seiten 2400 - 2403,
American Chemical Society, Easton, PA., US;
H. Ohta et al.: "Enantiotopically selective oxidation of alpha,omega-diols with the enzyme
systems of microorganisms"

(73) Patentinhaber: Haarmann & Reimer GmbH
Postfach 1253
W-3450 Holzminden(DE)

(72) Erfinder: Gatfield, Ian, Dr.
Ulmenweg 23
W-3470 Höxter(DE)
Erfinder: Sand, Theodor, Dr.
Vogelsang 3
W-3450 Holzminden(DE)

(74) Vertreter: Müller, Gerhard, Dr. et al
BAYER AG Konzernverwaltung RP Patentabteilung
W-5090 Leverkusen 1 Bayerwerk(DE)

## Beschreibung

Die vorliegende Erfindung betrifft ein neues Verfahren zur Herstellung von aliphatischen (acyclischen) Carbonsäuren durch mikrobielle Oxidation von aliphatischen (acyclischen) Alkoholen.

Es ist bekannt, Essigsäure durch mikrobielle Oxidation von verdünntem Ethanol mittels Luftsauerstoff herzustellen. Als Mikroorganismen werden bei dieser Herstellung Bakterien der Spezies Acetobacter verwendet (siehe z.B. Ullmanns Encyclopädie der technischen Chemie, 4. Auflage, Band 11, Seiten 41 ff.). Es ist ferner bekannt, daß Fuselalkohole, d.h. Alkohole mit höherer Kohlenstoffzahl und gegebenenfalls verzweigter C-Kette toxisch sind gegenüber Essigbakterien und deshalb nicht oder nur langsam und in schlechten Ausbeuten mikrobiell zu den entsprechenden Carbonsäuren oxidiert werden. So zeigen Ley & Kersters, Bacteriol. Rev. 28, 164 (1964), daß die Geschwindigkeiten, mit der die mikrobiellen Oxidationen von Isobutylalkohol und Propanol verlaufen, im Verhältnis 3:100 stehen. In "Comprehensive Biotechnology" Vol. 3, Herausgeber; Murray Moo-Young, erschienen in Pergamon Press 1985 stellt F.H.Sharpell im Kapitel 47, "Mikrobial Flavors and Fragrances" auf Seite 971 fest, daß die mikrobielle Oxidation von Isopentylalkohol zwar theoretisch möglich ist, daß aber bislang kein Mikroorganismus bekannt ist, der Isopentylalkohol tatsächlich zu 3-Methylbuttersäure zu oxidieren vermag.

H. Ohta et al., J. Org. Chem. 47, 2400-2404 (1982) beschreiben die Oxidation von 2-substituierten Propandiolen-1,3 und von 3-substituierten Pentandiolen-1.5 mit Hilfe von Gluconobacter roseus. Die Ergebnisse sind stark Substrat-abhängig: Die Oxidation führt zu Hydroxycarbonsäuren, Lactonen und - im Falle linealer Diole - auch zu Dicarbonsäuren. Eine Anregung zur mikrobiellen Oxidation einwertiger Alkohole, wie sie z.B. in Fuselölen vorkommen, konnte daraus nicht abgeleitet werden.

Überraschenderweise wurde jetzt gefunden, daß sich auch Fuselalkohole unter milden Bedingungen und ohne Zusatz von teuren und ausgefallenen Fermentationszusätzen mikrobiell zu den entsprechenden Carbonsäure oxidieren lassen, wenn man eine bestimmte Bakterien-Spezies, nämlich die Spezies Gluconobacter, verwendet. Mit Hilfe dieser Bakterien-Spezies gelingt es, Fuselalkohole, d.h. Alkohole mit längeren gegebenenfalls verzweigten C-Ketten in hohen Geschwindigkeiten und ausgezeichneten Ausbeuten, zu den entsprechenden Carbonsäuren zu oxidieren.

Gegenstand der Erfindung ist also ein Verfahren zur Herstellung aliphatischer Carbonsäuren aus der Reihe n-Buttersäure, Isobuttersäure, 2-Methylbuttersäure, 3-Methylbuttersäure (Isovaleriansäure), Geraniumsäure durch mikrobielle Oxidation von aliphatischen Alkoholen aus der Reihe n-Butanol, Isobutanol, 2-Methylbutanol, Isoamylalkohol, Geraniol mittels Luftsauerstoff, dadurch gekennzeichnet, daß man als Mikroorganismus Bakterien der Spezies Gluconobacter verwendet.

Für die Anwendung im erfindungsgemäßen Verfahren eignen sich alle im Handel befindlichen Stämme von Gluconobacter roseus; als Beispiele für solche im Handel befindlichen Stämme seien genannt:
der in der "List of Cultures" (1984) des Institute of Fermentation Osaka/Japan mit der Nummer IFO 3990 und der im Katalog des Institute of Applied Microbiology University of Tokyo/Japan mit der Nummer IAM 1841 bezeichnete Stamm.

Das erfindungsgemäße Verfahren wird vorzugsweise wie folgt ausgeführt:
Zunächst wird der erfindungsgemäß zu verwendende Mikroorganismus Gluconobacter roseus in einem üblichen Kulturmedium in für die Kultivierung von Mikroorganismen üblichen Weise kultiviert. Sobald im Kulturmedium eine ausreichende Keimzahl vorliegt, wird dieses mit dem zu oxidierenden Alkohol versetzt. Die Menge an Alkohol wird dabei vorteilhaft so bemessen, daß die Alkoholkonzentration 0,1 bis 25 g/l Kulturbrühe, vorzugsweise 1,5 bis 20 g/l Kulturbrühe beträgt. Nach beendeter Oxidation wird die Carbonsäure durch Ansäuern der Kulturbrühe auf einen pH-Wert von 3 bis 1, Extraktion mit einem geeigneten Lösungsmittel, z.B. Essigester und Abdestillieren des Lösungsmittels, gewonnen.

Die Kultivierung des erfindungsgemäß zu verwendenden Mikroorganismus Gluconobacter roseus kann in synthetischen, halbsynthetischen und natürlichen Kulturmedien erfolgen. Zur Herstellung des Kulturmediums können Glucose, Sucrose, Mannit, Sorbit, Glycerin, Dextrin, Stärke, vegetabile Öle usw. als Kohlenstoffquellen und Fleischextrakt, Peptone, Klebermehl, Baumwollsaatmehl, Sojabohnenmehr, Erdnußmehl, Fischmehl, Kornschlempe, Trockenhefe, Hefeextrakt, Ammoniumsulfat, Ammoniumnitrat, Harnstoff und andere organische und anorganische Stickstoffquellen als Stickstoffspender Verwendung finden. Ebenso können, falls erforderlich, Metallsalze, z.B. Sulfate, Nitrate, Chloride, Carbonate, Phosphate usw. von Natrium, Kalium, Magnesium, Calcium, Zink und Eisen dem Kulturmedium zugesetzt werden. Weiterhin kann das Kulturmedium mit Entschäumungsmitteln, z.B. Siliconöl, oberflächenaktive Mittel usw. enthalten.

Die Kultivierungstemperatur liegt gewöhnlich zwischen 18 und 40° C, vorzugsweise bei etwa 30° C. Gute Ergebnisse werden erhalten, wenn der pH-Wert des Kulturmediums konstant auf einem pH-Wert von 3,5 bis 7, vorzugsweise auf 4 bis 4,5 gehalten wird. Die Einstellung des pH-Wertes kann z.B. durch Zugabe von 25 %iger wäßriger Ammoniaklösung erfolgen. Die Kultivierung kann entweder in geeigneten Schüttelapparaturen oder in Fermentern mit Rühreinrichtung erfolgen; bei der Kultivierung ist für eine ausreichende Belüftung Sorge zu tragen.

Das erfindungsgemäße Verfahren eignet sich besonders zur Herstellung von Isobuttersäure, Isovaleriansäure und 2-Methylbuttersäure.

Die nach dem erfindungsgemäßen Verfahren herstellbaren aliphatischen Carbonsäuren sind Geschmacksstoffe oder können durch Umsetzung, z.B. durch Veresterung, in ebenfalls als Geschmacksstoffe verwendbare Derivate umgewandelt werden (siehe z.B. "Comprehensive Biotechnology" loc. cit.).

Beispiel 1

Eine Lösung aus 5 g Hefeextrakt, 3 g Fleischpepton und 25 g Mannit in 1 l Wasser wurde 20 Minuten bei 121 °C sterilisiert. Nach dem Abkühlen wurden 300 ml dieser Lösung mit Gluconobacter roseus angeimpft und in einem 1 l-Erlenmeyerkolben 2 Tage bei 30 °C geschüttelt. Dann wurde das Kulturmedium mit 0,5 g Isobutanol versetzt und weitere 3 Tage bei 30 °C geschüttelt.

Zum Aufarbeiten wurde die Kulturbrühe angesäuert, mit Essigester extrahiert und aus dem Extrakt das Lösungsmittel abdestilliert.

Es wurden 0,44 g einer Flüssigkeit erhalten, die ausweislich der gaschromatographischen Analyse zu 91 % aus Isobuttersäure bestand.

Beispiel 2

Es wurde wie in Beispiel 1 verfahren mit den Abänderungen, daß bei der Herstellung des Kulturmediums anstelle der 25 g Mannit die gleiche Menge Sorbit verwendet wurde und daß die Substratkonzentration auf 10 g Isobutanol/l Kulturbrühe erhöht wurde.

Der Gehalt an Isobuttersäure betrug nach 3 Tagen 12 g Isobuttersäure/l Fermentationslösung (bestimmt durch quantitative Hochdruckflüssigkeitschromatographie (HPLC)).

Beispiel 3

Ein 30 l-Fermenter wurde mit 21 l Wasser, 500 g Mannit, 100 g Hefeextrakt, 60 g Fleischextrakt und 5 g Antischaummittel beschickt und 20 Minuten bei 121 °C sterilisiert. Nach dem Abkühlen wurde die Lösung mit 500 ml einer Vorkultur, die wie in Beispiel 1 beschrieben hergestellt worden war, angeimpft und bei 28 °C, einer Belüftung mit 15 l Luft/Minute und einer Rührgeschwindigkeit von 300 Umdrehungen/Minute fermentiert. Nach 27 Stunden war der pH-Wert des Kulturmediums von 5,7 auf 4,2 gefallen und die Keimzahl von $7 \times 10^6$/ml Fermentationslösung auf $6 \times 10^9$/ml Fermentationslösung gestiegen. Diese Fermentationslösung wurde mit 315 g Isobutanol, entsprechend einer Substratkonzentration von 15 g/l, versetzt, die Belüftung auf 2 l Luft/Minute herabgesetzt und die Fermentation weitergeführt. Nach 15 Stunden war der pH-Wert der Fermentationsbrühe auf 3,8 abgesunken. Die HPLC-Analyse der angesäuerten Fermentationslösung ergab eine Isobuttersäurekonzentration von 14 g/l.

Eine 4 l-Probe der Fermentationsbrühe wurde mit konzentrierter Schwefelsäure auf pH 2 angesäuert und mit Essigester extrahiert. Nach dem Entfernen des Lösungsmittels aus dem Extrakt wurden 48 g einer Flüssigkeit erhalten, die gemäß gaschromatographischer Analyse zu 86 % aus Isobuttersäure bestand.

Beispiel 4

Es wurde wie in Beispiel 3 beschrieben verfahren, mit den Abänderungen, daß die Belüftung 12 l Luft/Minute und die Kultivationszeit 24 Stunden betrug. Die so erhaltene Vorkultur wurde zum Animpfen eines 300 l-Fermenters benutzt, der mit 180 l Wasser, 4,5 kg Mannit, 900 g Hefeextrakt, 540 g Fleischextrakt und 50 g Antischaummittel beschickt, sterilisiert und wieder auf Raumtemperatur abgekühlt war. Die so erhaltene Kulturbrühe wurde 25 Stunden bei 28 °C, einer Belüftung von 120 l Luft/Minute und einer Rührgeschwindigkeit von 100 Umdrehungen/Minute fermentiert. Während dieser Zeit fiel der pH-Wert der Fermentationslösung von 5,5 auf 4,1 und die Keimzahl stieg von $8 \times 10^8$/ml Fermentationslösung auf $4 \times 10^9$/ml Fermentationslösung. Die so erhaltene Fermentationsbrühe wurde mit 2,5 kg Isobutanol, entsprechend einer Substratkonzentration von 12,5 g/l, versetzt, die Belüftung auf 22 l Luft/Minute herabgesetzt und weitere 20 Stunden fermentiert; in dieser Zeit fiel der pH-Wert auf 3,8. Die Konzentration an Isobuttersäure in der Fermentationslösung wurde mit Hilfe der HPLC ermittelt und betrug nach 3 Stunden 6 g/l und nach 20 Stunden 15 g/l.

Beispiel 5

In einem 30 l Fermenter wurde nach der in Beispiel 3 beschriebenen Arbeitsweise ein Kulturmedium mit einer Keimzahl von $9 \times 10^9$/ml Kulturlösung und einem pH-Wert von 4,2 hergestellt. Dieses Kulturmedium wurde mit 420 g Isobutanol, entsprechend einer Substratkonzentration von 20 g/l versetzt und unter den in Beispiel 3 beschriebenen Bedingungen fermentiert; bei dieser Fermentation wurde jedoch der pH-Wert durch Zugabe 25 %iger wäßriger Ammoniaklösung auf dem Wert von 4,2 gehalten. Nach 15 Stunden wurde in der Fermentationslösung die Konzentration an Isobuttersäure mit Hilfe der HPLC bestimmt; sie betrug 21 g Isobuttersäure/l Fermentationslösung.

Eine 4 l Probe der Fermentationslösung wurde angesäuert und mit Essigester extrahiert. Nach dem Abdestillieren des Lösungsmittels aus dem Extrakt wurden 65 g einer Flüssigkeit erhalten, die nach gaschromatographischer Analyse zu 94 % aus Isobuttersäure bestand.

Beispiel 6

Es wurde wie in Beispiel 3 beschrieben verfahren nur wurden statt der 315 g Isobutanol nunmehr 210 g 2-Methylbutanol, entsprechend einer Substratkonzentration von 10 g/l, angewendet. Die Konzentration an 2-Methylbuttersäure in der Fermentationslösung wurde nach 24 Stunden mit Hilfe der HPLC ermittelt; sie betrug 7 g 2-Methylbuttersäure/l.

Zur Aufarbeitung wurde die Fermentationslösung mit konzentrierter Schwefelsäure auf pH 2 angesäuert und mit Essigester extrahiert. Nach dem Abdestillieren des Lösungsmittels aus dem Extrakt wurden 160 g einer Flüssigkeit erhalten, die gemäß gaschromatographischer Analyse zu 72 % aus 2-Methylbuttersäure bestand.

Beispiel 7

Es wurde wie in Beispiel 6 beschrieben verfahren, nur wurden statt der 210 g 2-Methylbutanol nunmehr 210 g Isoamylalkohol, entsprechend einer Substratkonzentration von 10 g/l eingesetzt.

Nach 15-stündiger Fermentation betrug die Konzentration an 3-Methylbuttersäure/l Fermentationslösung gemäß HPLC 10 g/l; nach 39-stündiger Fermentation war die Konzentration an 3-Methylbuttersäure auf 11 g/l angestiegen.

Die Aufarbeitung der gesamten Fermentationslösung ergab 178 g einer gelben Flüssigkeit, die gemäß gaschromatographischer Analyse zu 90 % aus 3-Methylbuttersäure bestand.

Beispiel 8

Es wurde wie in Beispiel 1 beschrieben verfahren, mit dem Unterschied, daß 500 ml des Nährmediums mit 5 g n-Butanol, entsprechend einer Substratkonzentration von 10 g/l eingesetzt wurden.

Die HPLC-Analyse der Fermentationslösung ergab eine Produktkonzentration von 13 g n-Buttersäure/l Fermentationslösung.

Beispiel 9

0,5 g Geraniol wurden 5 Tage unter den in Beispiel 1 angegebenen Bedingungen fermentiert. Zum Aufarbeiten der Fermentationslösung wurde diese auf einen pH-Wert von 2 angesäuert und mit Essigester extrahiert. Nach dem Abdestillieren des Lösungsmittels aus dem Extrakt wurden 0,41 g einer gelben Flüssigkeit erhalten, die gemäß gaschromatographischer Analyse 50 % Geraniumsäure enthielt.

Beispiel 10

Es wurde auf die in Beispiel 2 beschriebene Weise ein Kulturmedium hergestellt dessen pH-Wert jedoch durch Zugabe von wäßriger Ammoniaklösung konstant auf 4,5 gehalten wurde. So erhaltenes Kulturmedium wurde mit 158 g Isoamylalkohol versetzt und weitere 158 g Isoamylalkohol kontinuierlich innerhalb von 8 Stunden zudosiert. Nach 25-stündiger Fermentation ergab die HPLC-Analyse eine Produktkonzentration von 17 g 3-Methylbuttersäure/l Fermentationslösung.

**Patentansprüche**

1. Verfahren zur Herstellung aliphatischer Carbonsäuren aus der Reihe n-Buttersäure, Isobuttersäure, 2-Methylbuttersäure, 3-Methyl-buttersäure (Isovaleriansäure), Geraniumsäure durch mikrobielle Oxidation von aliphatischen Alkoholen aus der Reihe n-Butanol, Isobutanol, 2-Methylbutanol, Isoamylalkohol, Geraniol mittels Luftsauerstoff, dadurch gekennzeichnet, daß man als Mikroorganismus Bakterien der Spezies Gluconobacter roseus verwendet.

**Claims**

1. Process for the preparation of aliphatic carboxylic acids from the series consisting of n-butyric acid, isobutyric acid, 2-methylbutyric acid, 3-methyl-butyric acid (isovaleric acid) and geranic acid by microbial oxidation of aliphatic alcohols from the series consisting of n-butanol, isobutanol, 2-methylbutanol, isoamyl alcohol and geraniol by means of atmospheric oxygen, characterised in that bacteria of the species Gluconobacter roseus are used as the microorganism.

**Revendications**

1. Procédé de fabrication d'acides carboxyliques aliphatiques de la série acide n-butyrique, acide isobutyrique, acide méthyl-2-butyrique, acide méthyl-3-butyrique (acide isovalérique), acide géranique, par oxydation microbienne d'alcools aliphatiques de la série n-butanol, isobutanol, méthyl-2-butanol, alcool isoamylique, géraniol, au moyen de l'oxygène atmosphérique, caractérisé en ce qu'on utilise comme micro-organisme des bactéries de l'espèce Gluconobacter roseus.